# EUROPEAN PATENT SPECIFICATION

(11) **EP 1 432 406 B1**
(45) Date of publication and mention of the grant of the patent: **18.04.2007**
(21) Application number: 02751559.2
(22) Date of filing: 08.08.2002
(51) Int. Cl.: A61K 9/113, A61K 9/18

(54) **PHARMACEUTICAL COMPOSITION COMPRISING A WATER/OIL/WATER DOUBLE MICROEMULSION INCORPORATED IN A SOLID SUPPORT**
PHARMAZEUTISCHE ZUSAMMENSETZUNG ENTHALTEND EINE WASSER/ÖL/WASSER DOPPELTE MIKROEMULSION IN EINEM FESTEN TRÄGER
COMPOSITION PHARMACEUTIQUE COMPRENANT UNE DOUBLE MICRO-EMULSION EAU/HUILE/EAU

(30) Priority: 09.08.2001 IT MI20011756
(43) Date of publication of application: 30.06.2004
(73) Proprietor: Geotech Pharma Inc., Toronto, M5H 3S5 (CA)
(72) Inventor: CARLI, Fabio, I-34136 Trieste (IT); CHIELLINI, Elisabetta, I-56126 Pisa (IT)
(74) Representative: Gervasi, Gemma
(86) International application number: PCT/IB2002/003172
(87) International publication number: WO 2003/013421

(56) References cited:
- WO-A-00/09093
- WO-A-91/18613
- SHIVELY M L ET AL: "Oral bioavailability of vancomycin solid-state emulsions" INTERNATIONAL JOURNAL OF PHARMACEUTICS, vol. 117, no. 1, 1995, pages 119-122, XP002243773 ISSN: 0378-5173
- MYERS S L ET AL: "Preparation and characterization of emulsifiable glasses: Oil-in-water and water-in-oil-in-water emulsions" JOURNAL OF COLLOID AND INTERFACE SCIENCE, vol. 149, no. 1, 1992, pages 271-278, XP008018119 ISSN: 0021-9797

## Description

### Prior art

As described in French patent No. 2594693, water/oil (w/o) single emulsions containing a drug are transformed into "solid emulsions" through adsorption of the same by a hydrophilic-hydrophobic silica mixture. The resulting water-soluble drugs are slow-releasing type and, therefore, may be potentially used as controlled release forms (A.Berthod *et al.,* J.Pharm. Sci., 77, 216-221, 1988).

Water/oil (w/o) or oil/water (o/w) single microemulsions loaded on adsorbent powders are described e.g. in patent WO 00/09093. Out of their many applications, said microemulsions are suitable for the oral administration of drugs that-due to their polarity-can be little absorbed as they are scarcely permeable through the gastrointestinal mucous membrane.

Water/oil (w/o) single microemulsions, aimed at increasing the oral absorption of little permeable drugs, are also described in patent WO 9003164. In this case, the liquid microemulsion is formulated in the solid form as it is loaded on polymers and granulated.

However, said solid emulsions and microemulsions exhibit some unsatisfactory features. By way of example, their permeability through the gastrointestinal mucous membrane increases but is still insufficient for most drugs. Furthermore, the drug cannot be loaded in large amounts on the solid support it follows that a considerable active ingredient dilution is needed, which arouses problems in terms of size and volume of the final pharmaceutical form.

WO 91/18613 A discloses self-emulsifying glasses prepared from water soluble matrix compounds and e.g. a water in-oil-in-water emulsion.

### Summary of the invention

It has surprisingly been found that the problems encountered in the prior art are solved by using the pharmaceutical composition according to the present invention.

Said pharmaceutical composition comprises a water/oil/water (w/o/w) double microemulsion incorporated in a solid support. The internal aqueous phase of said microemulsion contains a dissolved drug.

The preferred drugs according to the present invention are the drugs exhibiting low permeability through the gastrointestinal mucous: membrane, more preferably the drugs having a polypeptidic/proteic structure and those having a high polarity or a high molecular weight.

The solid support consists of a microporous inorganic substance or of a colloidal inorganic adsorbent substance or of a cross-linked polymer.

With said composition, the gastrointestinal mucous membrane is extremely permeable to the drug; furthermore, the drug can be loaded in high amounts on the solid support.

### Brief description of the figures

Figure 1 shows the w/o/w microemulsion adsorption index by silica as per Example 1. The grams of the adsorbed w/o/w microemulsion per 100 g silica are plotted on the ordinate axis.
Figure 2 and Figure 3 have the same meaning as Figure 1, referred to Examples 3 and 4, respectively.
Figure 4 shows the adsorption index of the w/o/w microemulsion by silica as per Example 5.
Figure 5 shows the adsorption index of the w/o/w microemulsion by silica as per Example 3 (curve a) in comparison with the adsorption index of the w/o microemulsion (curve b) by the same type of silica.
Figure 6 represents the size of the oily microdrops released from the compositions of Example 1 (curve a) and of Example 3 (curve b), in a buffer solution, pH 7.4, at 37°C.
Figure 7 shows the permeability *ex vivo* on the rat of acyclovir from the composition of Example 1 (curve a) in comparison with acyclovir as it is (curve b).
Figure 8 shows the permeability *ex vivo* on the rat of acyclovir from the w/o composition of Example 3 (curve a) in comparison with a w/o composition incorporated in the same type of silica (curve b).

### Detailed description of the invention

The characteristics and advantages of the pharmaceutical composition comprising a water/oil/water double microemulsion incorporated in a solid support according to the present invention will be apparent from the detailed description that follows.

The microemulsion of the invention may be prepared according to a procedure consisting in the following steps:
a) drug dissolution in water;
b) addition of the solution as per a) to an oil;
c) addition of a surfactant/cosurfactant to the mixture as per b) and stirring with formation of the w/o microemulsion;
d) addition of the microemulsion as per c) to a water/surfactant/cosurfactant mixture and stirring with formation of the w/o/w double microemulsion.

The resulting w/o/w microemulsion is incorporated in the solid support to give the composition according to the present invention.

Minor modifications to this process may be effected, while the composition characteristics are kept constant.

As may be inferred from the process description, the w/o/w double microemulsion consists of water, oil, surfactant and cosurfactant, and the internal aqueous phase contains a dissolved drug.

Said double microemulsion shows the following composition by weight of the aqueous and oily phases:

| | |
|---|---|
| Water in the internal phase (w/o) | 0.5 to 5.0% |
| Oil | 3.0 to 10.0% |
| Surfactant | 2.0 to 15% |
| Cosurfactant (w/o internal phase) | 0 to 2.0% |
| Cosurfactant (w/o/w external phase): | 1.0 to 10% |
| Water in the external phase (w/o/w) | 50 to 90% |

Preferred drugs according to the present invention are the drugs soluble in water and poorly permeable through the gastrointestinal mucous membrane, in particular the drugs having a proteic/polypeptidic structure and those having a high polarity and a high molecular weight.

Said drugs are e.g. calcitonine, heparin, acyclovir, antibiotics, such as cephalosporins, insulin, interpheron, erythropoietin, doxorubicin, adriamycin, growth hormone, and somatotropin.

The w/o microemulsion aqueous phase, characterised in that the drug is dissolved, may be deionised water or a buffer solution at different pH values (1; 2; 5.5 and 7.5).

The microemulsion oily phase may be an oil of vegetable origin, such as olive oil, soya bean oil, maize oil, coconut oil, or of synthetic origin, such as esters of fatty acids, e.g. isopropylmiristate, isopropylpalmitate. Fatty acids may have a short or medium or long chain.

Also mixtures of mono-, di- and triglycerides of vegetable or synthetic or semisynthetic origin or polyethoxylated derivatives thereof may be used.

The surfactants used in the microemulsion must be non-toxic, of synthetic or of natural origin. Surfactant combinations having different chemical characteristics may be also used.

The surfactants particularly suitable for the invention are Tween®, Brij®, Cremophor®, Span®, Myverol®. The surfactants used in the preparation of the w/o/w microemulsion may be of synthetic origin, such as for example short-chain alcohols, e.g. ethanol, isopropanol, or of natural origin, such as for example lecithins, phospholipids or derivatives thereof.

The solid support consists of microporous inorganic substances or of high-surface-area colloidal inorganic adsorbent substances or of cross-linked polymers.

The microporous inorganic substances are selected from the group consisting of silica, silicates, zeolites, aluminas, activated carbons.

The adsorbent colloidal inorganic substances are selected from the group consisting of colloidal silica, magnesium trisilicate, clays, magnesium hydroxide, talc.

The cross-linked polymers are selected from the group consisting of crospovidone, cross-linked sodium carboxymethyl starch, cross-linked sodium carboxymethyl cellulose, cross-linked polystyrene, cross-linked polymethyl methacrylate.

In the w/o microemulsion the water/oil ratio ranges from 0.7 to 0.2 by weight.

In the w/o microemulsion the water/surfactant ratio ranges from 0.50 to 1.5 by weight. In the water/surfactant/cosurfactant mixture used in d), the water/surfactant ratio ranges from 8:1 to 12:1 by weight.

In the water/sunfactant/cosurfactant mixture used in d), the water/cosurfactant ratio ranges from 9:1 to 15:1 by weight.

The ratio of the water/surfactant/cosurfactant mixture to the w/o microemulsion used in d) ranges from 8:1 to 13:1 by weight.

The ratio of the w/o/w double microemulsion to the solid support ranges from 1:10 to 15:1 by weight.

The drug content in the final composition ranges from 1 ppm to 50% by weight.

The w/o microemulsion is prepared at a temperature of 20°C to 65 °C.

The w/o/w double microemulsion is prepared at a temperature of 20°C to 55°C.

The incorporation of the w/o/w double microemulsion is performed at a temperature of 20°C to 55°C, by means of high-efficiency apparatus, such as mixers, granulators and extruders.

The resulting composition is in the form of free-flowing powder having 0.1 to 800 µm particle size or in the form of a plastic mass workable by extrusion and spheronising, or by addition of further suitable excipients.

The compositions according to the present invention exhibit some important features:
1. The oily microdrops released from the solid support into an aqueous medium are less than 1 micrometer in size.
2. The drug dissolution rate in an appropriate aqueous buffer can be adjusted from a few-minute immediate release to a some-hour delayed release.
3. The permeability of little permeable drugs through the gastrointestinal mucous membrane increases considerably.

Said compositions for therapeutic use are prepared in the form of capsules or sachets or tablets or suspensions containing a pharmaceutically effective amount of drug and pharmaceutically acceptable excipients or diluents, if any.

The following examples referred to the preparation and characterisation of the claimed compositions are conveyed to illustrate the present invention.

### Example 1

| Double microemulsion (w/o/w) components | Quantity (g) |
|---|---|
| Isopropylmiristate | 0.846 |
| Lecithin Lipoid S-40® | 0.282 |
| Tween 80 | 2.30 |
| Acyclovir | 0.005 |
| Buffer, pH 1.4 | 0.517 |
| Water | 23.1 |
| Ethanol | 2.31 |

A w/o microemulsion was prepared by mixing, under magnetic stirring at 25°C, an isopropylmiristate/lecithin solution (3:1) as an oily phase, a buffer solution, pH 1.4, containing dissolved acyclovir as an aqueous phase (0.517 g), and surfactant Tween 80 (0.8 g).

The w/o microemulsion was added under stirring (600 rpm for 100 min) to a water/ethanol/Tween 80 mixture (15:1.5:1.0) in a microemulsion/(water/ethanol/Tween 80) ratio of 0.09:1 to give the w/o/w double microemulsion containing acyclovir.

The resulting w/o/w double microemulsion was incorporated in colloidal silica Aerosil 300® by granulation in a high-efficiency granulator (Battagion, Bergamo, Italy).

The ratio of the w/o/w microemulsion to silica was equal to 7.6:1 by weight.

The composition obtained was in the form of free-flowing powder having a uniform particle size.

### Example 2

| Double microemulsion (w/o/w) components | Quantity (g) |
|---|---|
| Isopropylmiristate | 1.66 |
| Lecithin Lipoid S-40® | 0.550 |
| Sodium heparin | 5000 I.U. |
| Water (internal phase) | 1.034 |
| Tween 80 | 4.51 |
| Water (external phase) | 46.2 |
| Ethanol | 4.53 |

A w/o microemulsion was prepared by mixing at 25°C an isopropylmiristate/lecithin solution (3:1) as an oily phase, water (1.034 g) containing heparin, and surfactant Tween 80 (1.5 g).

The mixture was maintained under magnetic stirring at 600 rpm for 1 hr.

The w/o microemulsion was added to a water/ethanol/Tween 80 mixture (15:1.5:1.0) in a microemulsion/(water/ethanol/Tween 80) ratio of 0.09:1.

The mixture was maintained under magnetic stirring at 600 rpm for 1 hr to give the w/o/w double microemulsion.

The resulting w/o/w double microemulsion was incorporated in cross-linked crospovidone polymer, Kollidon CL®, in a twin screw extruder APV. Finally the product was spheronised.

The ratio of the w/o/w microemulsion to crospovidone was equal to 1:1 by weight.

The free-flowing powder obtained had a uniform particle size.

### Example 3

| Double microemulsion (w/o/w) components | Quantity (g) |
|---|---|
| Akoline® | 0.672 |
| Acyclovir | 0.127 |
| Water | 13.9 |
| Tween 80 | 0.470 |
| Buffer, pH 1.4 | 0.277 |

A w/o microemulsion was prepared by mixing, under magnetic stirring at 35°C, Akoline® as an oily phase, a buffer solution, pH 1.4, containing dissolved acyclovir as an aqueous phase (0.227 g), and surfactant Tween 80 (0.470 g).

The w/o microemulsion was added under stirring (600 rpm for 60 min) to a water/ethanol mixture (10:1.0) in a (water/ethanol)/microemulsion ratio of 9.9:1 to give a w/o/w double microemulsion containing acyclovir.

The resulting w/o/w double microemulsion was incorporated in colloidal silica in a 7.7:1 ratio.

The composition obtained was in the form of free-flowing powder having a uniform particle size.

### Example 4

| Double microemulsion (w/o/w) components | Quantity |
|---|---|
| Labrasol® | 1.60 g |
| Tween 80 | 1.90 g |
| Calcitonine | 2200 I.U. |
| Acid aqueous solution | 41.3 g |
| Water | 41.3 g |
| Ethanol | 4.1 g |

A w/o microemulsion was prepared at 25°C by addition of Labrasol/Tween 80 solution (2.9:1) to the 0.1N HCl acid aqueous solution containing calcitonine (2200 I.U./ml).

The mixture was maintained under magnetic stirring at 600 rpm for 40 min, added with the remainder of the Tween 80, and allowed to stir at 600 rpm for 1 hr.

The resulting w/o microemulsion was added to a water/Tween 80/ethanol solution in a microemulsion/(water/Tween 80/ethanol) ratio of 0.101:1. The mixture was allowed to stir at 450 rpm for 30 min, and then at 500 rpm for 60 min. A w/o/w microemulsion was obtained.

The microemulsion was incorporated in colloidal silica by means of a high-efficiency granulator, in an emulsion/colloidal silica ratio of 12.5:1 by weight.

### Example 5

| Double microemulsion (w/o/w) components | Quantity (g) |
|---|---|
| Isopropylmiristate | 0.846 |
| Lecithin Lipoid S-40® | 0.282 |
| Tween 80 | 2.30 |
| Acyclovir | 0.005 |
| Buffer, pH 1.4 | 0.517 |
| Water | 23.1 |
| Ethanol | 2.31 |

A w/o microemulsion was prepared by mixing, under magnetic stirring at 25°C, an isopropylmiristate/lecithin solution (3:1) as an oily phase, a buffer solution, pH 1.4, containing dissolved acyclovir as an aqueous phase (0.517 g), and surfactant Tween 80 (0.8 g).

The w/o microemulsion was added under stirring (600 rpm for 100 min) to a water/ethanol/Tween 80 mixture (15:1.5:1.0) in a microemulsionl(waterlethanoU Tween 80) ratio of 0.09:1 to give the w/o/w double microemulsion containing acyclovir.

The w/o/w double microemulsion obtained was incorporated in microporous silica Syloid® by granulation in a high-efficiency granulator (Battagion, Bergamo, Italy).

The ratio of the w/o/w microemulsion to silica was equal to 5.0:1 by weight.

The resulting composition was in the form of powder.

### Characterisation tests

The compositions of the present invention were characterised by the following procedures.

### Adsorption index

The index of adsorption of the w/o/w microemulsion and the w/o microemulsion as per Example 1 by silica as per Example 1 was determined using a fritted bottom cylinder.

The amount of liquid adsorbed by silica was determined gravimetrically by the difference between the weight of the powder after adsorption and the weight of silica as it is.

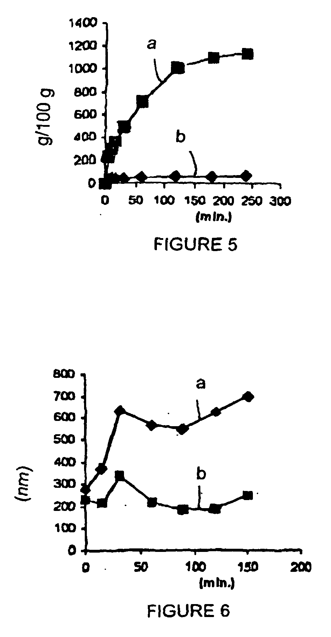

The adsorption index of the w/o/w double microemulsion is higher than that of the w/o microemulsion (Figure 5).

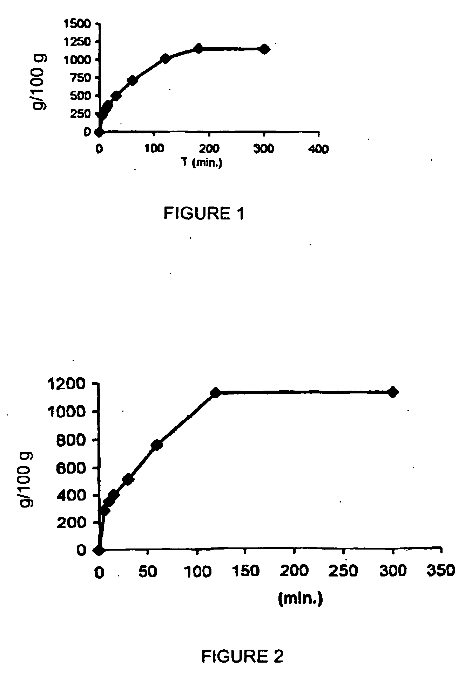

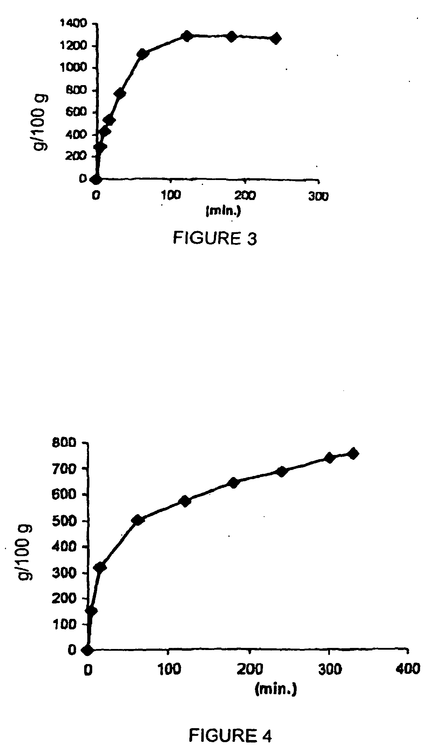

The adsorption index of the w/o/w microemulsion by colloidal silica increases over time and can vary depending on the type of the basic oily mixture used (Figures Nos. 1, 2, and 3). The adsorption index of a double microemulsion by microporous silica is shown in Figure 4.

### Size of the microdrops released from the compositions of the invention

The size of the microdrops released from the compositions as per Examples 1 and 3 was determined by dispersing the composition under magnetic stirring in a buffer solution, pH 7.4, at 37°C. Samples of 4 ml each were taken from the suspension at 0, 15, 30, 60, 90, 120, 150 min intervals. After centrifuging, the supernatant was analysed by the Laser Light Scattering technique. The microdrops size ranged from 100 to 700 nm (Figure 6).

### In vitro release test

The amount of acyclovir released over time from the compositions as per Examples 1 and 5 was determined by suspending the composition in 900 ml buffer, pH 7.4, under stirring (100 rpm at 37°C). At predetermined times, adequate samples were taken and centrifuged. The supernatant was analysed by HPLC.

**Table 1 - Acyclovir release rate from the compositions as per Examples 1 and 5**

| Time (hr) | % release-Example 1 | % release-Example 5 |
|---|---|---|
| 0.15 | 95.0 | 2.0 |
| 1.0 | 99.5 | 7.5 |
| 6.0 | 100.0 | 64.0 |

The data of Table 1 show that the release rates differ considerably depending on the types of silica used as a solid support (colloidal silica as per Example 1 and microporous silica as per Example 5).

### Ex vivo permeation tests

The *ex vivo* permeation tests were performed on Wistar rats weighing about 250-300 g, fasted for approx. 8 hrs prior to the experiment. The animal was anaesthetized by ethyl urethane injected i.m. (1.5 mg/kg bodyweight).

The peritoneum was opened and the intestinal tract, from the Treitz ligament to approx. mid-jejunum, was isolated. The isolated intestinal tract was cannulated by two cannulas, for the perfusate inlet and outlet, respectively. Once the isolated intestinal tract was washed with a buffer solution, pH 7.4, it was perfused with a suspension containing the active ingredient in a concentration of 120 γ/ml of the composition as per Example 1, in a buffer, pH 7.4. The recycle method was used.

Samples of 0.1 ml were taken from the mother suspension at various intervals (0, 15, 30, 60 min), centrifuged and analysed by HPLC.

The active ingredient absorption through the intestinal wall was calculated by the difference between the value of the initial concentration and the value of the recycling suspension at the various time intervals.

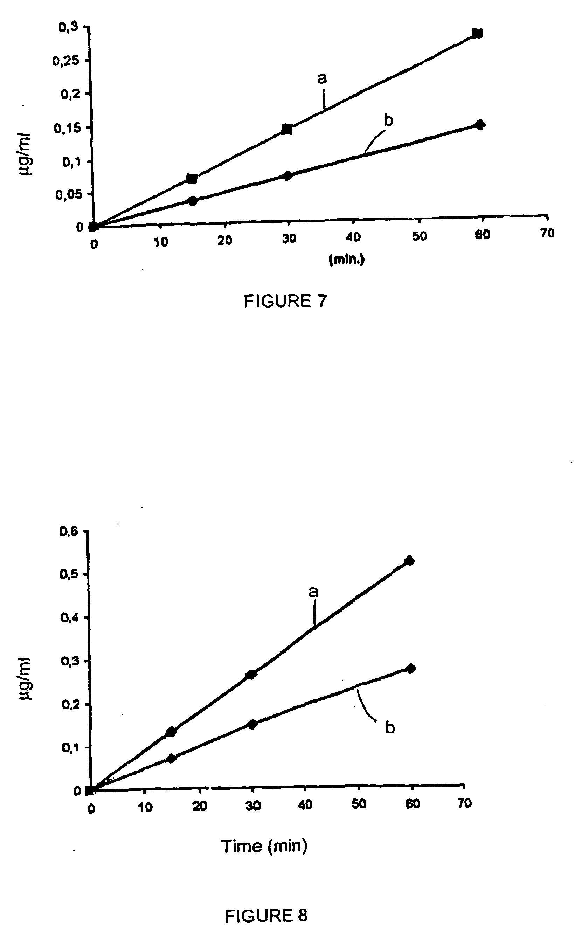

The permeability found in the case of the suspensions obtained with the w/o/w double microemulsion in colloidal silica is considerably higher than that of the active ingredient as it is (Figure 7).

Furthermore, the data shown in Figure 8 prove that, using the same colloidal silica as a solid support, the permeability that may be obtained with a w/o/w double microemulsion according to the invention is much higher than that obtainable with a single w/o microemulsion.

## Claims

1. Pharmaceutical composition comprising a water/oil/water double microemulsion whose aqueous phase contains a dissolved drug, **characterised in that** said double microemulsion is incorporated in a solid support selected from a microporous inorganic substance, a colloidal adsorbent substance and a cross-linked polymer
wherein:
said microporous inorganic substance is selected from the group consisting of silica, silicates, zeolites, aluminas and activated carbons;
said colloidal adsorbent substance is selected from the group consisting of colloidal silica, magnesium trisilicate, clays, magnesium hydroxide, and talc;
said cross-linked polymer is selected from the group consisting of crospovidone, cross-linked sodium carboxymethyl starch, cross-linked sodium carboxymethyl cellulose, cross-linked polystyrene, cross-linked polymethyl methacrylate.

2. The composition as claimed in claim 1, **characterised in that** said double microemulsion consists of water (w/o internal phase) in a quantity of 0.5 to 5.0%, oil in a quantity of 3.0 to 10%, a surfactant in a quantity of 2.0 to 15%, and a cosurfactant (w/o internal phase) in a quantity of 0 to 2.0%, water (w/o/w external phase) in a quantity of 50 to 90%, a cosurfactant (w/o/w external phase) of 1.0 to 10%, all percentages being by weight of the aqueous and oily phases.

3. The composition as claimed in claim 1, **characterised in that** the ratio of said microemulsion to said solid support ranges from 1:10 to 15:1 by weight.

4. The composition as claimed in claim 1, **characterised in that** the drug content ranges from 1 ppm to. 50.0% by weight.

5. The composition as claimed in claim 1, **characterised in that** said drug is selected from the group comprising calcitonine, heparin, acyclovir, cephalosporins, insulin, interpheron, erythropoietin, doxorubicin, adriamycin, growth hormone, and somatotropin.

6. The composition as claimed in claim 2, **characterised in that** said oil is selected from the group comprising olive oil, soya bean oil, maize oil, coconut oil, isopropylmiristate, isopropylpalmitate, and mono-, di- and triglycerides mixtures or polyethoxylated derivatives thereof.

7. The composition as claimed in claim 2, **characterised in that** said surfactant is selected from the group consisting of Tween®, Brij®, Cremophor®, Span®, Myverol®.

8. The composition as claimed in claim 2, **characterised in that** said cosurfactant is selected from the group consisting of ethanol, isopropanol, lecithins and phospholipids or derivatives thereof.

9. Double microemulsion (w/o/w) containing a dissolved drug in the internal aqueous phase as claimed in claim 1, having the following by wt. composition of the aqueous and oily phases:
| | |
|---|---|
| Water of the internal phase (w/o) | 0.5 to 5.0% |
| Oil | 3.0 to 10.0% |
| Surfactant | 2.0 to 15.0% |
| Cosurfactant (w/o internal phase) | 0 to 2.0% |
| Cosurfactant (w/o/w external | 1.0 to 10.0% |
| phase) | |
| Water of the external phase | 50 to 90.0% |

## Patentansprüche

1. Pharmazeutische Zusammensetzung, umfassend eine Wasser-Öl-Wasser-Doppelmikroemulsion, deren wässrige Phase ein gelöstes Arzneimittel enthält, **dadurch gekennzeichnet, dass** die Doppelmikroemulsion in einen festen Träger eingearbeitet ist, der gewählt ist aus einer mikroporösen anorganischen Substanz, einer kolloidalen Adsorbens-Substanz und einem vernetzten Polymer, worin
die mikroporöse anorganische Substanz gewählt ist aus der Gruppe, die besteht aus Siliciumoxid, Silicaten, Zeolithen, Aluminiumoxiden und Aktivkohlen;
die kolloidale Adsorbens-Substanz gewählt ist aus der Gruppe, die besteht aus kolloidalem Siliciumoxid, Magnesiumtrisilicat, Tonen, Magnesiumhydroxid und Talkum;
das vernetzte Polymer gewählt ist aus der Gruppe, die besteht aus Crospovidone, vernetzter Natriumcarboxymethyl-Stärke, vernetzter Natriumcarboxymethyl-Cellulose, vernetztem Polystyrol, vernetztem Polymethylmethacrylat.

2. Zusammensetzung nach Anspruch 1, **dadurch gekennzeichnet, dass** die Doppelmikroemulsion aus Wasser (w/o-Innen-Phase) in einer Menge von 0,5 bis 5,0 %, Öl in einer Menge von 3,0 bis 10 %, einem oberflächenaktiven Mittel bzw. Tensid in einer Menge von 2,0 bis 15 % und einem Co-Tensid (w/o-Innen-Phase) in einer Menge von 0 bis 2,0 %, Wasser (w/o/w-Außen-Phase) in einer Menge von 50 bis 90 % und einem Co-Tensid (w/o/w-Außen-Phase) in einer Menge von 1,0 bis 10 % besteht, wobei alle Prozentwerte Werte sind, die bezogen sind auf das Gewicht der wässrigen und Öl-Phasen.

3. Zusammensetzung nach Anspruch 1, **dadurch gekennzeichnet, dass** das Verhältnis der Mikroemulsion zu dem festen Träger in einem Bereich von 1:10 bis 15:1 liegt, angegeben in Bezug auf das Gewicht.

4. Zusammensetzung nach Anspruch 1, **dadurch gekennzeichnet, dass** der Arzneimittel-Gehalt im Bereich von 1 ppm bis 50,0 Gew.-% liegt.

5. Zusammensetzung nach Anspruch 1, **dadurch gekennzeichnet, dass** das Arzneimittel gewählt ist aus der Gruppe, die besteht aus Calcitonin, Heparin, Acyclovir, Cephalosporinen, Insulin, Interferon, Erythropoietin, Doxorubicin, Adriamycin, Wachstums-Hormon und Somatotropin.

6. Zusammensetzung nach Anspruch 2, **dadurch gekennzeichnet, dass** das Öl gewählt ist aus der Gruppe, die besteht aus Olivenöl, Sojabohnenöl, Maisöl, Kokosnussöl, Isopropylmyristat, Isopropylpalmitat und Mono-, Di- und Triglycerid-Mischungen oder poly-ethoxylierten Derivaten davon.

7. Zusammensetzung nach Anspruch 2, **dadurch gekennzeichnet, dass** das oberflächenaktive Mittel bzw. Tensid gewählt ist aus der Gruppe, die besteht aus Tween®, Brij®, Cremophor®, Span® und Myverol®.

8. Zusammensetzung nach Anspruch 2, **dadurch gekennzeichnet, dass** das Co-Tensid gewählt ist aus der Gruppe, die besteht aus Ethanol, Isopropanol, Lecithinen und Phospholipiden oder Derivaten davon.

9. Doppelmikroemulsion (w/o/w) enthaltend ein gelöstes Arzneimittel in der inneren wässrigen Phase nach Anspruch 1, das die folgende gewichtsbezogene Zusammensetzung der wässrigen und Öl-Phasen aufweist:
| | |
|---|---|
| Wasser der Innen-Phase (w/o) | 0,5 bis 5,0 % |
| Öl | 3,0 bis 10,0 % |
| oberflächenaktives Mittel bzw. Tensid | 2,0 bis 15,0 % |
| Co-Tensid (w/o-Innen-Phase) | 0 bis 2,0 % |
| Co-Tensid (w/o/w-Außen-Phase) | 1,0 bis 10,0 % |
| Wasser der Außen-Phase | 50 bis 90,0 % |

## Revendications

1. Composition pharmaceutique comprenant une double microémulsion eau/huile/eau dont la phase aqueuse contient un produit pharmaceutique dissous, **caractérisée en ce que** ladite double microémulsion est incorporée dans un support solide sélectionné parmi une substance inorganique microporeuse, une substance adsorbante colloïdale et un polymère réticulé, où
ladite substance inorganique microporeuse est sélectionnée dans le groupe consistant en silice, silicates, zéolites, alumines et carbones activés,
ladite substance adsorbante colloïdale est sélectionnée dans le groupe consistant en silice colloïdale, trisilicate de magnésium, argiles, hydroxyde de magnésium et talc ;
ledit polymère réticulé est sélectionné dans le groupe consistant en crospovidone, sodium carboxyméthylamidon réticulé, sodium carboxyméthylcellulose réticulée, polystyrène réticulé, polyméthyl méthacrylate réticulé.

2. Composition selon la revendication 1, **caractérisée en ce que** ladite double microémulsion consiste en eau (phase interne e/h) en une quantité de 0,5 à 5,0%, huile en une quantité de 3,0 à 10%, un agent tensioactif en une quantité de 2,0 à 15%, et un co-agent tensioactif (phase interne e/h) en une quantité de 0 à 2,0%, eau (phase externe e/h/e) en une quantité de 50 à 90%, un co-agent tensioactif (phase externe e/h/e) de 1,0 à 10%, tous les pourcentages étant en poids des phases aqueuses et dans l'huile.

3. Composition selon la revendication 1, **caractérisée en ce que** le rapport de ladite microémulsion audit support solide est compris entre 1 :10 et 15 :1 en poids.

4. Composition selon la revendication 1, **caractérisée en ce que** la teneur en produit pharmaceutique est comprise entre 1 ppm et 50,0% en poids.

5. Composition selon la revendication 1, **caractérisée en ce que** ledit produit pharmaceutique est sélectionné dans le groupe consistant en calcitonine, héparine, acyclovir, céphalosporines, insuline, interféron, érythropoïétine, doxorubicine, adriamycine, hormone de croissance, et somatotropine.

6. Composition selon la revendication 2, **caractérisée en ce que** ladite huile est sélectionnée dans le groupe consistant en huile d'olive, huile de soja, huile de mais, huile de noix de coco, isopropylmiristate, isopropylpalmitate, et mono-di et triglycerides en mélanges ou leurs dérivés polyoxyéthylés.

7. Composition selon la revendication 2, **caractérisée en ce que** ledit agent tensioactif est sélectionné dans le groupe consistant en Tween®, Brij®, Cremophor®, Span®, Myverol®.

8. Composition selon la revendication 2, **caractérisée en ce que** ledit co-agent tensioactif est sélectionné dans le groupe consistant en éthanol, isopropanol, lécithines et phospholipides ou leurs dérivés.

9. Double microémulsion (e/h/e) contenant un produit pharmaceutique dissous dans la phase aqueuse interne selon la revendication 1, ayant la composition en poids qui suit des phases aqueuses et dans l'huile :
| | |
|---|---|
| eau de la phase interne (e/h/e) | 0,5 à 5,0% |
| huile | 3,0 à 10,0% |
| agent tensioactif | 2,0 à 15,0% |
| co-agent tensioactif (e/h phase interne) | 0 à 2,0% |
| co-agent tensioactif (e/h/e phase externe) | 1,0 à 10,0% |
| eau de la phase externe | 50 à 90,0%. |
